# EUROPEAN PATENT APPLICATION

(11) **EP 2 256 499 A1**
(43) Date of publication of application: **01.12.2010**
(21) Application number: 09706609.6
(22) Date of filing: 28.01.2009
(51) Int. Cl.: G01N 33/66, G01N 33/96, G01N 27/62

(54) **METHOD OF TESTING RHEUMATOID ARTHRITIS BY ANALYZING SUGAR CHAIN**

(30) Priority: 29.01.2008 JP 2008017498; 20.05.2008 JP 2008131885
(71) Applicant: National University Corporation Hokkaido University, Sapporo-shi, Hokkaido 060-0808 (JP); Shionogi & Co., Ltd., Osaka 541-0045 (JP)
(72) Inventor: NISHIMURA, Shin-Ichiro, Sapporo-shi Hokkaido 001-0021 (JP); MINAMI, Akio, Sapporo-shi Hokkaido 060-8638 (JP); FURUKAWA, Jun-ichi, Sapporo-shi Hokkaido 001-0021 (JP); SHINOHARA, Yasuro, Sapporo-shi Hokkaido 001-0021 (JP)
(74) Representative: White, Nina Louise
(86) International application number: PCT/JP2009/051316
(87) International publication number: WO 2009/096403

(57) **Abstract**

Provided is a novel method of diagnosing rheumatoid arthritis. Based on the quantitative expression profile of sugar chain expression amount in the serum (whole serum, HAP or LAP), the relevancy thereof to rheumatoid arthritis is analyzed. As a result, a sugar chain and a glycoprotein showing a change depending on the onset of rheumatoid arthritis are found out and thus a serum sugar chain and a glycoprotein usable as a novel biomarker are provided.

## Description

### TECHNICAL FIELD

The present invention relates to a method for diagnosing a rheumatoid arthritis using a sugar chain analysis.

### BACKGROUND ART

Rheumatoid arthritis (RA) is a chronic inframmatory disease wherein a synovial tissue is a principal lesion area and its prevalence rate makes up 1 % of the population. RA brings about synovitis in the early stages and then, cartilage and bones are gradually invaded and with progression, joints are destroyed and thereby deformed. It is known that an increased level of many proteinases, which break down proteoglycan and collagen in a cartilage tissue, such as matrix metalloproteinase (MMP), catepsin, peptidase and the like (see nonpatent documents 1 and 2). In addition, it is known that interleukin-1β (IL-1β), tumor necrosis factor-α (TNF-α) as well as various cytokines and enzyme inhibitors also play an important role in pathogenesis of RA (see nonpatent documents 3 and 4).
These proteins deteriorate a disease condition such that chronic inframmation is brought to destruction of a joint while implicating a knee and a joint region. For example, a nonsteroidal antiinflammatory drug (NSAID), a disease modifying anti-rheumatic drug (DMARD) and a biological agent (an anti-TNF receptor and an IL-1 antagonist) are used in drug therapy of a RA patient (see nonpatent documents 5-7). However, many of the RA patients do not recover from their disease conditions and continue to feel pain and to suffer bone destruction in spite of hard effort for treatment. Early diagnosis and early treatment are important also for the point of improving the quality of life of a RA patient.

As described above, RA is a disease which can result in a serious condition. The current diagnostic approach is based on "Revised ACR standard of classification in RA" established by American College of Rheumatology (ACR). The standard is composed of the following items:
1) morning stiffness lasting more than 1 hour (mainly in fingers);
2) swelling in more than 3 joints;
3) swelling in joints of hand (wrists, metacarpophalangeal joints and proximal interphalangeal joints);
4) swelling in symmetrical joints (right and left joints);
5) abnormal findings of radiography of hand;
6) subcutaneous nodules; and
7) test positive for rheumatism by a blood test.
The case which satisfies more than 4 items is diagnosed as RA

The blood test of the above item 7) measures mainly a rheumatoid factor (RF) in a serum. RF is an autoantibody against an Fc part of IgG which is frequently expressed in a serum of a RA patient. RF from a healthy individual is a polyactive antibody which reacts with various proteins other than IgG. However, RF from a RA patient includes a monoactive antibody, which reacts only with IgGFc, in addition to the polyactive antibody. This monoactive antibody has a 100 times higher activity to IgGFc than that of a polyactive antibody. Therefore, said RF is commonly measured as an index of determination of a RA patient.

However, there also exists a seronegative RA patient that RF is not detected from her serum at any time. In addition, it is known that some healthy individuals (about 2 %) have a positive reaction. Usefulness of an autoantibody other than a rheumatoid factor in early diagnosis has also been reported. For example, an anti-Sa protein antibody, an anti-perinuclear factor, an anti-ceratin antibody, an anti-filagrin antibody and an anti-cyclic citrullinated peptide (anti-CCP) antibody are known as such an autoantibody (see nonpatent document 8). Although an anti-CCP antibody is detected in about 76 % of serums from RA patients, it is also detected in about 4 % of serums from patients with rheumatoid conditions who are not determined as a RA patient (see nonpatent document 9 and 10). It is pointed out that an epitope with citrulline is involved in pathogenesis of RA because a citrullinated protein is detected in a synovial membrane from a RA patient (see nonpatent documents 11 and 12). However, the epitope with citrulline remain to be fully elucidated.

In addition, for example, a method wherein urine from a RA patient is subjected to a liquid chromatography and the patient is judged as a RA patient based on the presence of a peak of the component specific for RA in the chromatography (see patent document 1); and a method for diagnosing RA based on a change of sugar chain composition by cleaving a sugar chain from a glycoprotein, preparing an analysis sample by purification after labeling the sugar chain, and then, analyzing the sample with a high-performance liquid chromatography using ODS-silica column for obtaining the sugar chain composition of the sample (see patent document 2) are proposed. However, these methods require cumbersome procedures for preparing an analysis sample and did not have a satisfactory sensitivity or specificity.

[Patent document 1] JP-A-H07-72133
[Patent document 2] JP-A-H08-228795
[Nonpatent document 1] Landewe R. et al., Arthritis Rheum, 2004, Vol.50, p. 1390-1399)
[Nonpatent document 2] Tchetverikov I, An Rheum. Dis., 2003, Vol.62, p.1094-1099
[Nonpatent document 3] Cross A, et al., Arthritis Rheum. 2004, Vol.50, p.1430-1436
[Nonpatent document 4] Raap T., et al., J. Rheumatol., 2000, Vol.27, p.2558-2565
[Nonpatent document 5] Klimiuk PA, et al., J. Rheumatol., 2004, Vol.31, p.238-242
[Nonpatent document 6] Dougados M, et al., J. Rheumatol., 2003, Vol.30, p.2572-2579

[Nonpatent document 7] De A, et al., J. Rheumatol., 2002, Vol.29, p.46-51
[Nonpatent document 8] Martinus A.M. et al., Arthritis Research (2002), 4(2), p.87-93
[Nonpatent document 9] Gerard A. Schellekens, et al., J. Clin. Invest, (1998), 101(1), p.273-281
[Nonpatent document 10] ZhiJie Zhou, and Henri-Andre Menard), Current Opinion in Rheumatology (2002) 14(3), p.250-253
[Nonpatent document 11] Walter J van Venrooij and Ger J.M. Pruijn), Arthritis Research, (2000), 2(4), p.249-251
[Nonpatent document 12] Christine Masson-Bessie, et al., J. Immunol., (2001), 166(6), p.4177-4184

### DISCLOSURE OF INVENTION

### PROBLEM TO BE SOLVED

Among the above mentioned diagnoses, the anti-CCP antibody is the most promising antibody because of its specificity and sensitivity. Nevertheless, the specificity remains at 76 %. Accordingly, development of a biomarker which can be used as single or in combination having more excellent sensitivity and higher specificity, which permits early diagnosis and prognostic prediction, has been required.

### MEANS FOR SOLVING PROBLEM

The present inventors performed a large-scale analysis of a serum of patient having an individual disease condition by applying a mass spectrometric technique such as MALDI-TOF MS to a serum sugar chain which was prepared by an existing high-speed exhaustive sugar chain enrichment technique [Nishimura, S, K Niikura, M Kurogochi, T Matsushita, M Fumoto, H Hinou, R Kamitani, H Nakagawa, K Deguchi, N Miura, K Monde, and H Kondo, "High-Throughput Protein Glycomics: Combined Use of Chemoselective Glycoblotting and Maldi-Tof/Tof Mass Spectrometry." Angew Chem Int Ed Engl 44, no. 1 (2004): 91-96]. Then, they established a diagnostic method with a high sensitivity and specificity by correlating the serum sugar chain of a patient to the individual disease condition based on an expression profile of the sugar chain, finding a sugar chain showing a change, and then identifying a serum sugar chain which become a new biomarker, a glycoprotein having the varying sugar chain level, or a group of molecules involved in a biosynthesis pathway of the sugar chain showing a change.

This invention relates to the following (1) to (19).
(1) A method for diagnosing rheumatoid arthritis using an expression level of one or more sugar chains as an index, wherein the sugar chains are selected from a group consisting of No. 1, 7, 8, 10, 11, 17, 18, 21, 24, 25, 27, 29, 31, 38, 41, 43, 45, 46 and 48.
(2) A method for diagnosing rheumatoid arthritis using an expression level of one or more sugar chains as an index, wherein the sugar chains are selected from a group consisting of No.7, 18, 25, 29, 38, 31, 43 and 45.
(3) The method according to the above invention (1) or (2), wherein the selected sugar chain is No. 45.
(4) The method according to any of inventions (1) to (3), wherein the index is an expression level of the sugar chains in a serum.
(5) Use of one or more sugar chains as a marker for rheumatoid arthritis, wherein the sugar chains are selected from a group consisting of No. 1, 7, 8, 10, 11, 17, 18, 21, 24, 25, 27, 29, 31, 38, 41, 43, 45, 46 and 48.
(6) Use of one or more sugar chains as a marker for rheumatoid arthritis, wherein the sugar chains are selected from a group consisting of No.7, 18, 25, 29, 31, 38, 43 and 45.
(7) The use according to the above invention (5) or (6), wherein the selected sugar chain is No. 45.
(8) A method for diagnosing the presence of rheumatoid arthritis in a subject, wherein the method comprises:
   a process of collecting a sample containing sugar chains from the subject; and
   a process of diagnosing the presence of rheumatoid arthritis in the subject using an expression level of one or more sugar chains as an index in the above sample, wherein the sugar chains are selected from a group consisting of No. 1, 7, 8, 10, 11, 17, 18, 21, 24, 25, 27, 29, 31, 38, 41, 43, 45, 46 and 48.
(9) A method for diagnosing the presence of rheumatoid arthritis in a subject, wherein the method comprises:
   a process of collecting a sample containing sugar chains from the subject; and
   a process of diagnosing the presence of rheumatoid arthritis in the subject using an expression level of one or more sugar chains as an index in the above sample, wherein the sugar chains are selected from a group consisting of No. 7, 18, 25, 29, 31, 38, 43 and 45.
(10) The method according to the above invention (8) or (9), wherein the selected sugar chain is No. 45.
(11) The method according to any of (8) to (10), wherein the sample is a serum.
(12) A method for analyzing whether an expression level of sugar chains is within the normal range in human, wherein the method comprises a process for measuring an expression level of one or more sugar chains in a sample collected from a subject, wherein the sugar chains are selected from a group consisting of No. 1, 7, 8, 10, 11, 17, 18, 21, 24, 25, 27, 29, 31, 38, 41, 43, 45, 46 and 48.
(13) A method for analyzing whether an expression level of sugar chains is within the normal range in human, wherein the method comprises a process for measuring an expression level of one or more sugar chains in a sample collected from a subject, wherein the sugar chains are selected from a group consisting of No. 7, 18, 25, 29, 31, 38, 41, 43 and 45.
(14) The method according to the above invention (12) or (13), wherein the selected sugar chain is No. 45.
(15) The method according to any of (12) to (14), wherein the sample is a serum.
(16) A method for screening a rheumatoid arthritis sample comprising the following processes:
   (i) analyzing the sugar chains in a test sample; and
   (ii) comparing an expression level of one or more sugar chains of a sample from a healthy individual with those of the test sample, wherein the sugar chains are selected from a group consisting of No. 1, 7, 8, 10, 11, 17, 18, 21, 24, 25, 27, 29, 31, 38, 41, 43, 45, 46 and 48.
(17) A method for screening a rheumatoid arthritis sample comprising the following processes:
   (i) analyzing sugar chains in a test sample; and
   (ii) comparing an expression level of one or more sugar chains of a sample from a healthy individual with those of the test sample, wherein the sugar chains are selected from a group consisting of No.7, 18, 25, 29, 38, 31, 43 and 45.
(18) The method according to the above invention (16) or (17), wherein the selected sugar chain is No. 45.
(19) The method according to any of (16) to (18), wherein the sample is a serum.

In addition, this invention relates to the following (1) to (7).
(1) A method for diagnosing rheumatoid arthritis using an expression level of a sugar chain No. 45 as an index, wherein a fucose in the sugar chain composition is attached to a side chain.
(2) A method for diagnosing rheumatoid arthritis using an expression level of a sugar chain as an index, wherein the sugar chain has the composition of No. 45 and has sialyl Lewis x (NeuNAcα2→3Galβ1-4(Fucα1→3)GlcNAc) or sialyl Lewis a (NeuNAcα2→3Galβ1→3(Fucα1→4)GlcNAc) as an epitope.
(3) Use of a sugar chain No. 45 as a rheumatoid arthritis marker, wherein a fucose in the sugar chain composition is attached to a side chain.
(4) Use of a sugar chain as a rheumatoid arthritis marker, wherein the sugar chain has the composition of No. 45 and has sialyl Lewis x (NeuNAcα2→3Galβ1→4(Fucα1→3)GlcNAc) or sialyl Lewis a (NeuNAcα2→3Galβ1→3(Fucα1→4)GlcNAc) as an epitope.
(5) A method for diagnosing rheumatoid arthritis using an expression level of hemopexin, haptoglobin, ceruloplasmin, clasterin, kininogen-1, α1-antichymotrypsin, apolipoprotein H, α2-HS glycoprotein, α1-acid glycoprotein-1, α1-acid glycoprotein-2, zinc α2-glycoprotein or plasma protease C1 inhibitor as an index, which is modified by a sugar chain No. 45.
(6) A method for diagnosing rheumatoid arthritis using an expression level of hemopexin, haptoglobin, ceruloplasmin, clasterin, kininogen-1, α1-antichymotrypsin, apolipoprotein H, α2-HS glycoprotein, α1-acid glycoprotein-1, α1-acid glycoprotein-2, zinc α2-glycoprotein or plasma protease C1 inhibitor as an index, which is modified by a sugar chain in which a fucose in the sugar chain composition of No. 45 is attached to a side chain.
(7) A method for diagnosing rheumatoid arthritis using an expression level of hemopexin, haptoglobin, ceruloplasmin, clasterin, kininogen- 1 , α1-antichymotrypsin, apolipoprotein H, α2-HS glycoprotein, α1-acid glycoprotein-1, α1-acid glycoprotein-2, zinc α2-glycoprotein or plasma protease C 1 inhibitor as an index, which is modified by a sugar chain having the composition of No. 45 and having sialyl Lewis x (NeuNAcα2→3Galβ1→4(Fucα1→3)GlcNAc) or sialyl Lewis a (NeuNAcα2→3Galβ1→3(Fucα1→4)GlcNAc) as an epitope.

### EFFECT OF INVENTION

According to the present invention, a method for diagnosing rheumatoid arthritis which has higher sensitivity and specificity than those of a conventional method, or has complementary superiority is provided, and thereby, early diagnosis of a rheumatoid arthritis patient becomes possible. In addition, it becomes possible to provide a proper medical treatment by introducing the early diagnosis, and therefore, it can be expected that the method can contribute to the improvement of therapeutic effect and prognosis.

### BEST MODE FOR CARRYING OUT THE INVENTION

According to the present invention, all of N-type sugar chains in a blood are recovered from a serum of the subject by Glycoblotting and the quantitative profiles of them are obtained by MALDI-TOF MS. With addition of a known amount of an oligosaccharide as an internal standard, an amount of the sugar chains other than the internal standard in the serum can be easily calculated based on the area of the internal standard on the spectrum. For example, the detectable oligosaccharides are shown in the following Table 1.

**[Table 1] Detected N-linked Sugar Chain**

| No. | Composition |
|---|---|
| 1 | (Hex)₂ + (Man)₃(GlcNAc)₂ |
| 2 | (Hex)₁ (HexNAc)₁ + (Man)₃(GlcNAc)2 |
| 3 | (HexNAc)₂ + (Man)₃(GlcNAc)₂ |
| 4 | (Hex)₃ + (Man)₃(GlcNAc)₂ |
| 5 | (HexNAc)₂ (Deoxyhexose)₁ + (Man)₃(GlcNAc)₂ |
| 6 | (Hex)₁ (HexNAc)₂ + (Man)₃(GlcNAc)₂ |
| 7 | (HexNAc)₃ + (Man)₃(GlcNAc)₂ |
| 8 | (Hex)₄ + (Man)₃(GlcNAc)₂ |
| 9 | (Hex)₁ (HexNAc)₁ (NeuAc)₁ + (Man)₃(GlcNAc)₂ |
| 10 | (Hex)₁ (HexNAc)₂ (Deoxyhexose)₁ + (Man)₃(GlcNAc)₂ |
| 11 | (Hex)₂ (HexNAc)₂ + (Man)₃(GlcNAc)₂ |
| 12 | (HexNAc)₃ (Deoxyhexose)₁ + (Man)₃(GlcNAc)₂ |
| 13 | (Hex)₁ (HexNAc)₃ + (Man)₃(GlcNAc)₂ |
| 14 | (Hex)₅ + (Man)₃(GlcNAc)₂ |
| 15 | (Hex)₁ (HexNAc)₁ (Deoxyhexose)₁ (NeuAc)₁ + (Man)₃(GlcNAc)₂ |
| 16 | (Hex)₂ (HexNAc)₁ (NeuAc)₁ + (Man)₃(GlcNAc)₂ |
| 17 | (Hex)₁ (HexNAc)₂ (NeuAc)₁ + (Man)₃(GlcNAc)2 |
| 18 | (Hex)₂ (HexNAc)₂ (Deoxyhexose)₁ + (Man)₃(GlcNAc)₂ |
| 19 | (Hex)₁ (HexNAc)₃ (Deoxyhexose)₁ + (Man)₃(GlcNAc)₂ |
| 20 | (Hex)₂ (HexNAc)₃ + (Man)₃(GlcNAc)₂ |
| 21 | (Hex)₆ + (Man)₃(GlcNAc)₂ |
| 22 | (Hex)₃ (HexNAc)₁ (NeuAc)₁ + (Man)₃(GlcNAc)₂ |
| 23 | (Hex)₁ (HexNAc)₂ (Deoxyhexose)₁ (NeuAc)₁ + (Man)₃(GlcNAc)₂ |
| 24 | (Hex)₂ (HexNAc)2 (NeuAc)₁ + (Man)₃(GlcNAc)₂ |
| 25 | (Hex)₁ (HexNAc)₃ (NeuAc)₁ + (Man)₃(GlcNAc)₂ |
| 26 | (Hex)₂ (HexNAc)₃ (Deoxyhexose)₁ + (Man)₃(GlcNAc)₂ |
| 27 | (Hex)₂ (HexNAc)₂ (Deoxyhexose)₁ (NeuAc)₁ + (Man)₃(GlcNAc)₂ |
| 28 | (Hex)₁ (HexNAc)₃ (Deoxyhexose)₁ (NeuAc)₁ + (Man)₃(GlcNAc)₂ |
| 29 | (Hex)₂ (HexNAc)₃ (NeuAc)₁ + (Man)₃(GlcNAc)₂ |
| 30 | (Hex)₃ (HexNAc)₄ + (Man)₃(GlcNAc)₂ |
| 31 | (Hex)₂ (HexNAc)₂ (NeuAc)₂ + (Man)₃(GlcNAc)₂ |
| 32 | (Hex)₁ (HexNAc)₃ (Deoxyhexose)₂ (NeuAc)₁ + (Man)₃(GlcNAc)₂ |
| 33 | (Hex)₁ (HexNAc)₃ (NeuAc)₂ + (Man)₃(GlcNAc)₂ |
| 34 | (Hex)₂ (HexNAc)₃ (Deoxyhexose)₁ (NeuAc)₁ + (Man)₃(GlcNAc)₂ |
| 35 | (Hex)₃ (HexNAc)₃ (NeuAc)₁ + (Man)₃(GlcNAc)₂ |
| 36 | (Hex)₂ (HexNAc)₂ (Deoxyhexose)₁ (NeuAc)₂ + (Man)₃(GlcNAc)₂ |
| 37 | (Hex)₂ (HexNAc)₃ (NeuAc)₂ + (Man)₃(GlcNAc)₂ |
| 38 | (Hex)₂ (HexNAc)₃ (Deoxyhexose)₁ (NeuAc)₂ + (Man)₃(GlcNAc)₂ |
| 39 | (Hex)₃ (HexNAc)₃ (NeuAc)₂ + (Man)₃(GlcNAc)₂ |
| 40 | (Hex)₂ (HexNAc)₄ (NeuAc)₂ + (Man)₃(GlcNAc)₂ |
| 41 | (Hex)₃ (HexNAc)₃ (Deoxyhexose)₁ (NeuAc)₂ + (Man)₃(GlcNAc)₂ |
| 42 | (Hex)₂ (HexNAc)₄ (Deoxyhexose)₁ (NeuAc)₂ + (Man)₃(GlcNAc)₂ |
| 43 | (Hex)₃ (HexNAc)₃ (NeuAc)₃ + (Man)₃(GlcNAc)₂ |
| 44 | (Hex)₄ (HexNAc)₄ (NeuAc)₂ + (Man)₃(GlcNAc)₂ |
| 45 | (Hex)₃ (HexNAc)₃ (Deoxyhexose)₁ (NeuAc)₃ + (Man)₃(GlcNAc)₂ |
| 46 | (Hex)₄ (HexNAc)₄ (NeuAc)₃ + (Man)₃(GlcNAc)₂ |
| 47 | (Hex)₄ (HexNAc)₄ (Deoxyhexose)₁ (NeuAc)₃ + (Man)₃(GlcNAc)₂ |
| 48 | (Hex)₄ (HexNAc)₄ (NeuAc)₄ + (Man)₃(GlcNAc)2 |
| 49 | (Hex)₄ (HexNAc)₄ (Deoxyhexose)₁ (NeuAc)₄ + (Man)₃(GlcNAc)₂ |

[In the Table, "Man" represents mannose, "GlcNAc" represents N-acetylglucosamine, "Hex" represents hexose, and "NeuAc" represents N-acetylneuraminic acid.]

Then, a condition of rheumatoid arthritis is deduced from an expression level of one or more sugar chains, or from an increase or decrease of one or more sugar chains, and therefore, a method useful for making diagnosis, prediction and control of prognosis of rheumatoid arthritis can be obtained. For example, it is found that four sugar chains (No.7, 25, 29 and 38) having bisecting GlcNAc tend to increase in a rheumatoid arthritis patient. There are remarkably increased No. 43 having trisialyl structure and No. 45 attached by a fucose in a rheumatoid arthritis patient. In addition, a significantly increased expression level of a total amount of an N-linked sugar chain in serum and No. 31 which is a main sugar chain occurs in a rheumatoid arthritis patient. On the other hand, although it has been already reported that No. 5 tends to increase in a rheumatoid arthritis patient, No. 18 which is No. 5 attached by a galactose tends to be lower than it in a healthy individual.
In particular, No. 45 is a significant marker which makes possible to distinguish a healthy individual from a rheumatoid patient (P<0.0005). It is shown by the ROC curve that No. 45 is more excellent biomarker than RF which is an existing biomarker and that the usefulness of No. 45 is the same or more than an anti-CCP antibody which is currently recognized as the most useful antibody.

### Example 1

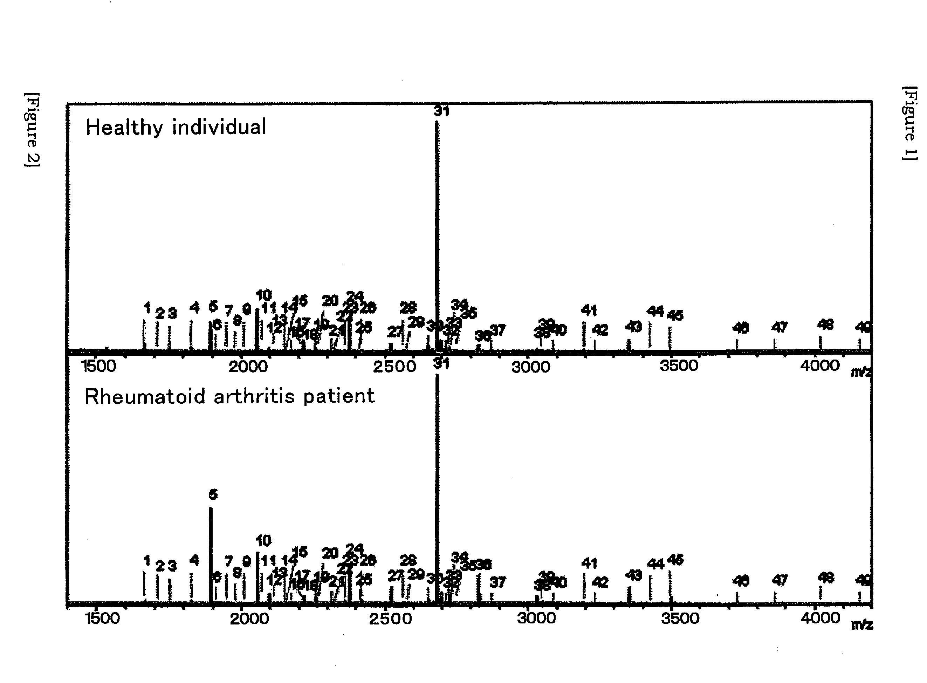

Sugar chains in serums from 14 rheumatoid arthritis patients and 11 healthy individuals were analyzed. This time, all of the serums used were collected from women and the average age of both groups was 56. All of the N-linked sugar chains in a serum are recovered by Glycoblotting (Nishimura et al.) and the quantitative profiles of them are obtained by MALDI-TOF MS.
MALDI spectrum of the N-linked sugar chains from the rheumatoid arthritis patients and the healthy individuals is shown in Figure 1. In addition, the expression levels of the sugar chains in the healthy individuals and the rheumatoid arthritis patients are shown in Figure 2. A quantitative value of a sugar chain calculated from an area ratio between the sugar chain and the known level of an oligosaccharide, which was added as an internal standard, on the spectrum is used as an expression level of each sugar chain. It was confirmed that there was a significant increase of an expression level of each sugar chain No.7, 25, 29, 31, 38, 43 and 45 and a significant decrease of an expression level of a sugar chain No. 18 in the rheumatoid arthritis patients. In addition, it was confirmed that there was a significant increase of the total amount of the N-linked sugar chains in a serum in the rheumatoid arthritis patients.

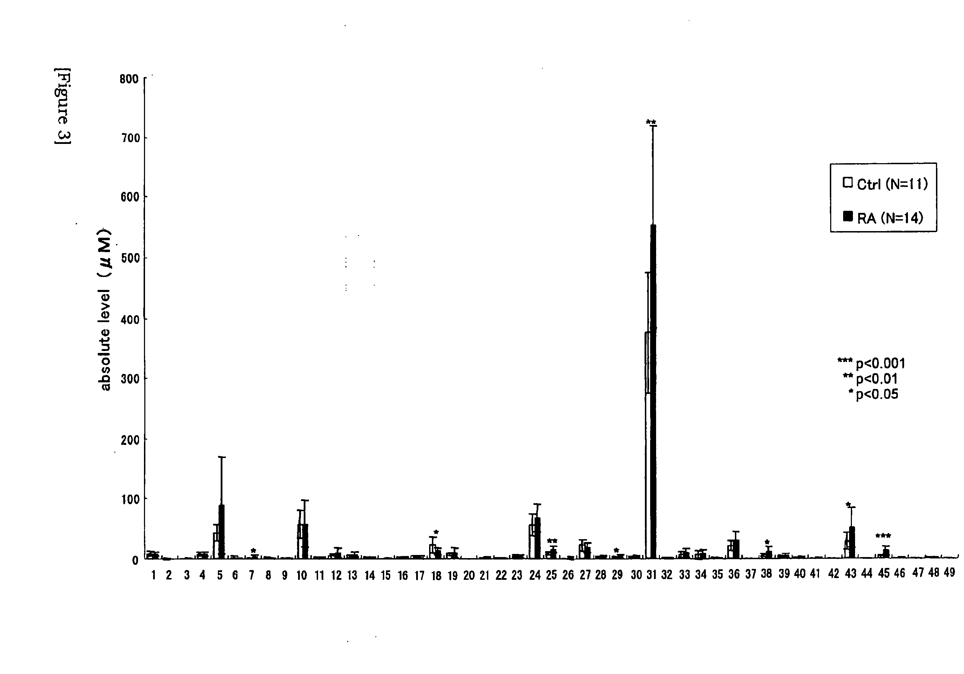

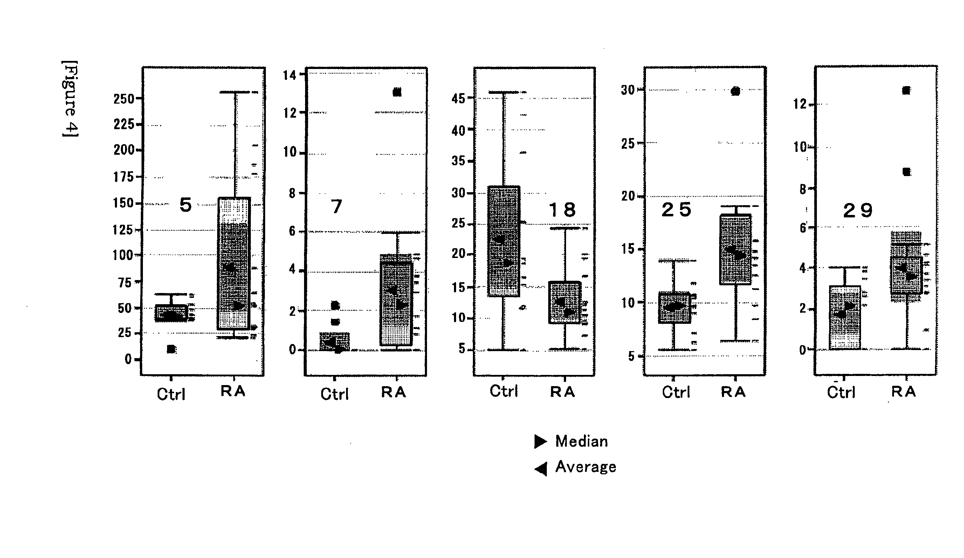

Each of Figure 3 and Figure 4 is a boxplot which shows a distribution of an expression level of the sugar chains in each sample of the healthy individuals and the rheumatoid arthritis patients, which was made by a statistical analysis software (SpotFire Decision Site for Functional Genomics, Spotfire, Inc.).

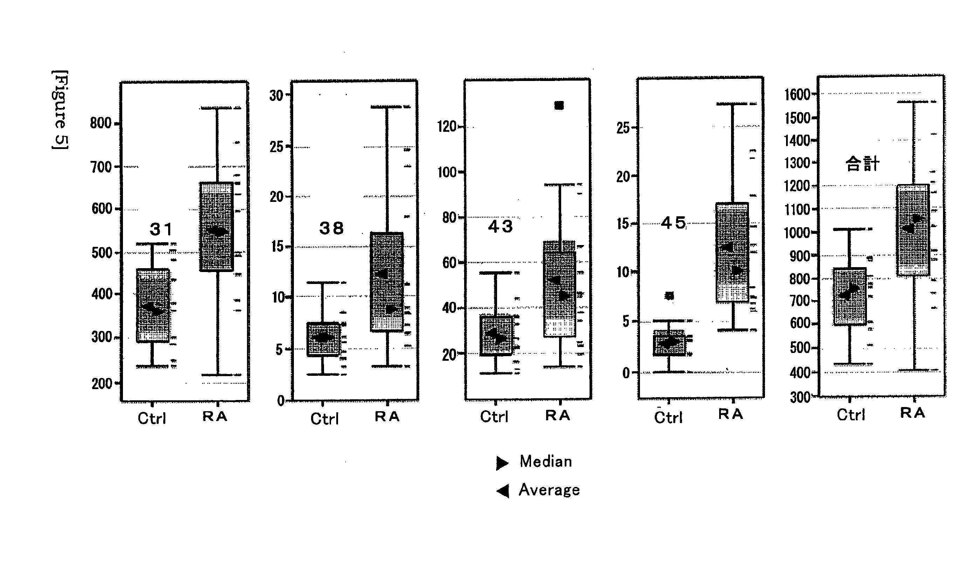

Figure 5 shows ROC curves of the existing RA biomarkers (RF, anti-CCP antibody and MMP-3) and No. 45.
As used herein, ROC is an abbreviation of "Receiver Operation Characteristic". In this example, a measurement is carried out for each target marker in two groups (patient and non-patient) and the sensitivity and the specificity were calculated with respect to each value of the measurement. The term "sensitivity" means "a ratio of a positive result in a group of patient" and the term "specificity" means "a ratio of a negative result in a group of non-patient ". The ROC curve is made by plotting "a value calculated by subtracting a specificity value from 1 (false positive, FP)" on the horizontal axis and "a sensitivity value (true positive, TP)" on the vertical axis, and then, connecting the plotted points. A marker which is plotted on an area where is nearer to the upper left corner is judged as an excellent marker which has less misdiagnosis and high reliability, that is, high diagnostic efficiency. This criterion is quantified by determining an AUC (area under the curve) value of the ROC curve. A marker which has an AUC value that is nearer to 1 (maximum value) is judged as an excellent marker. The AUC value of the three existing RA biomarkers and the sugar chain No. 45 are shown in Table 2 below.

**[Table 2]**

| Marker | | AUC |
|---|---|---|
| Existing RA biomarker | | 0.782 |
| | RF | |
| | Anti-CCP antibody | 0.898 |
| | MMP-3 | 0.688 |
| Sugar chain of the present invention | | |
| | Sugar chain No.45 | 0.955 |

As described above, No. 45 was a significant marker which makes possible to distinguish a healthy individual from a rheumatoid patient (P<0.0005). The ROC curve of No. 45 was more excellent than that of RF which is an existing biomarker. The usefulness of No. 45 was same or more than an anti-CCP antibody which was currently recognized as the most useful antibody.

### Example 2

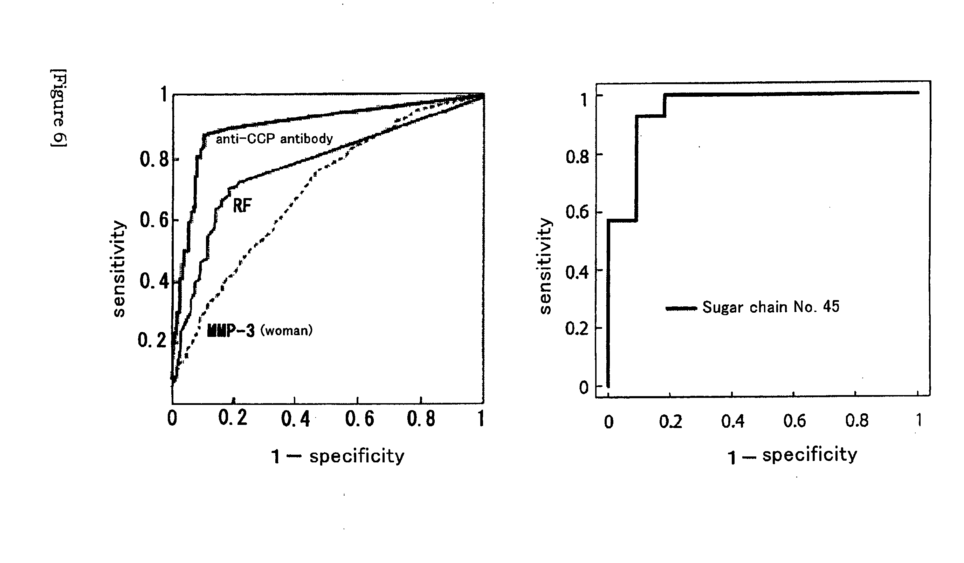

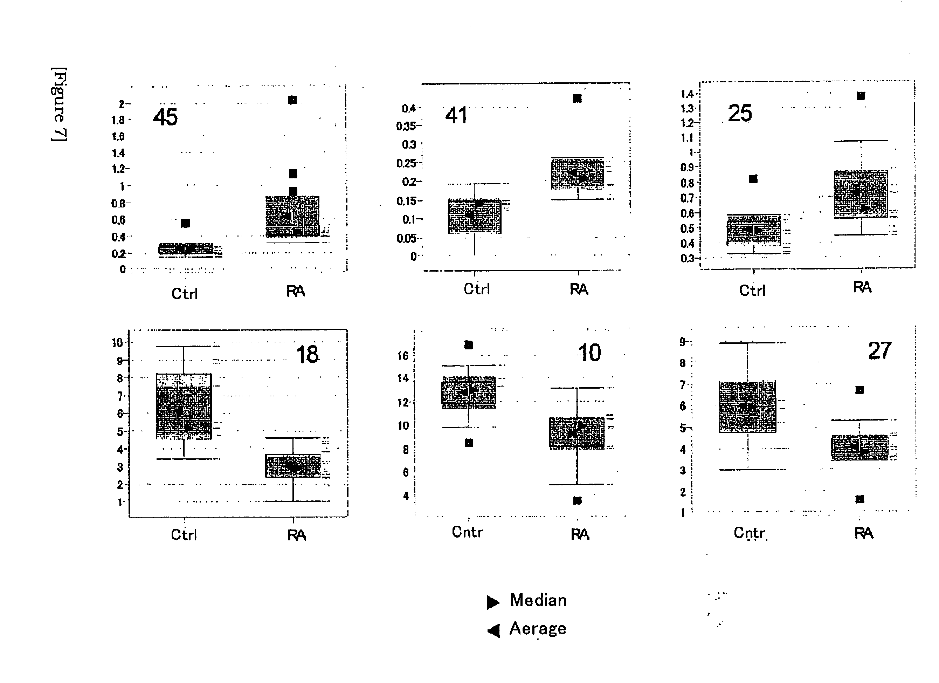

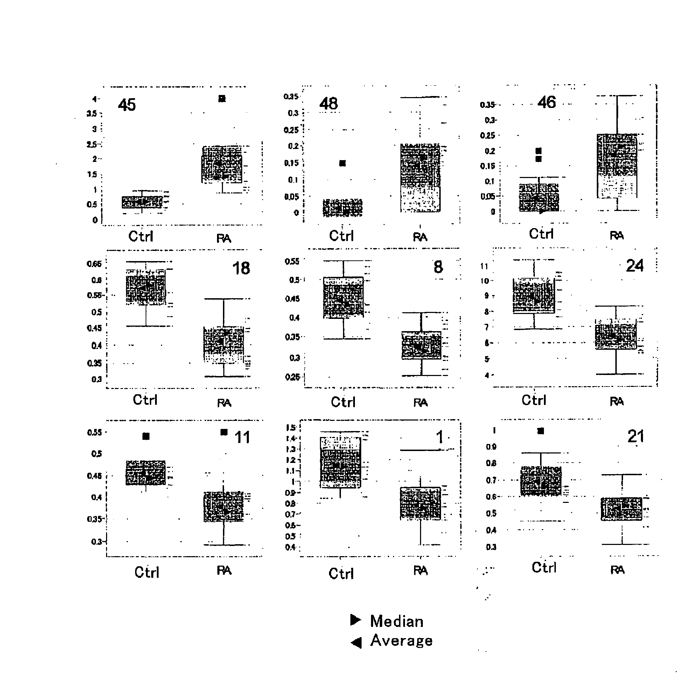

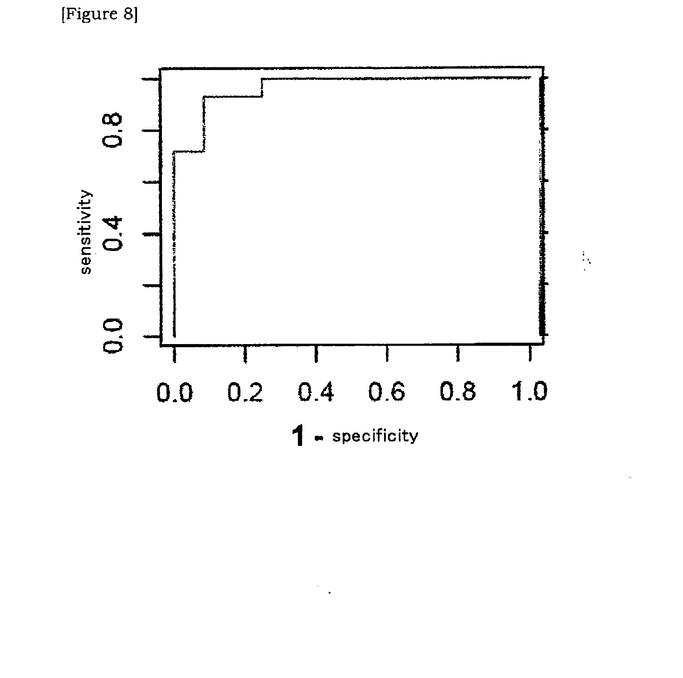

A fraction containing highly abundant proteins (HAP) and a fraction containing medium to low abundant proteins (LAP), which was made by removing HAP from the former fraction, were prepared by using an antibody column against the six highly abundant proteins in a serum (albumin, IgG, IgA, transferrin, antitrypsin and haptoglobin). Each N-linked sugar chains were recovered by Glycoblotting and the quantitative glycome profiles of them are compared between the healthy individuals and the rheumatoid arthritis patients by MALDI-TOF MS.
An expression level of each sugar chain was compared by using a relative value which was calculated from a ratio that was obtained by dividing an area of signals of an individual sugar chain on the spectrum by sum of the areas of the signals of all sugar chains detected. An expression level of an individual sugar chain was compared between the healthy individuals and the rheumatoid arthritis patients by using a two sided t-test. As a result, it was confirmed that there was a significant increase of an expression level of each sugar chain No.25, 41 and 45 (p<0.01) and a significant decrease of an expression level of each sugar chain No. 10, 18 and 27 (p<0.01) in HAP of the rheumatoid arthritis patients. It was confirmed that there was a significant increase of an expression level of each sugar chain No.45, 46 and 48 (p<0.01) and a significant decrease of an expression level of each sugar chain No.1, 8, 10, 11, 17, 18, 21 and 24 (p<0.01) in LAP of the rheumatoid arthritis patients. Each boxplot which shows a distribution of an expression level of the sugar chains having a significant variation of the expression level between the healthy individuals and the rheumatoid arthritis patients is shown in Figure 6 and Figure 7. An ROC curve of the sugar chain No. 45 having the most significant variation of the expression level along with a condition of rheumatoid arthritis in LAP is shown in Figure 8. An AUC of the ROC curve of the sugar chains having a significant variation of the expression level is shown in Table 3 below. In addition, each AUC of the ROC curve of the three existing RA biomarkers and the sugar chain No. 45 is shown in Table 4 below.

**[Table 3]**

| No. | HAP | | | LAP | | |
|---|---|---|---|---|---|---|
| | Detect. Rate* Normal | RA | AUC | Detect Rate* Normal | RA | AUC |
| 1 | 1.0 | 0.9 | 0.690 | 1.0 | 1.0 | 0.833 |
| 7 | 0.9 | 1.0 | 0.661 | 0.1 | 0.0 | NA |
| 8 | 1.0 | 1.0 | 0.685 | 1.0 | 1.0 | 0.940 |
| 10 | 1.0 | 1.0 | 0.827 | 1.0 | 0.9 | 0.827 |
| 11 | 1.0 | 1.0 | 0.774 | 1.0 | 1.0 | 0.881 |
| 17 | 0.7 | 1.0 | 0.750 | 1.0 | 1.0 | 0.810 |
| 18 | 1.0 | 1.0 | 0.952 | 1.0 | 1.0 | 0.964 |
| 21 | 1.0 | 0.9 | 0.750 | 1.0 | 1.0 | 0.851 |
| 24 | 1.0 | 1.0 | 0.619 | 1.0 | 1.0 | 0.911 |
| 25 | 1.0 | 1.0 | 0.804 | 0.3 | 0.5 | 0.634 |
| 27 | 1.0 | 1.0 | 0.827 | 1.0 | 1.0 | 0.750 |
| 29 | 1.0 | 1.0 | 0.619 | 0.3 | 0.6 | 0.619 |
| 31 | 1.0 | 1.0 | 0.819 | 1.0 | 1.0 | 0.613 |
| 38 | 1.0 | 1.0 | 0.792 | 0.3 | 0.5 | 0.577 |
| 41 | 0.8 | 1.0 | 0.881 | 0.7 | 0.9 | 0.714 |
| 43 | 1.0 | 1.0 | 0.726 | 1.0 | 1.0 | 0.804 |
| 45 | 1.0 | 1.0 | 0.905 | 1.0 | 1.0 | 0.964 |
| 46 | 0.0 | 0.1 | NA | 0.3 | 0.7 | 0.810 |
| 48 | 0.0 | 0.0 | NA | 0.1 | 0.6 | 0.801 |

| | | | | | | |
|---|---|---|---|---|---|---|
| *) Detection rate of each group | | | | | | |

**[Table 4]**

| Marker | | AUC |
|---|---|---|
| Existing RA biomarker | | 0.782 |
| | RF | |
| | Anti-CCP antibody | 0.898 |
| | MMP-3 | 0.688 |
| Sugar chain of the present invention | | 0.903 |
| | Sugar chain No.45 (Whole Serum) | 0.905 |
| | Sugar chain No.45 (HAP) | 0.964 |
| | Sugar chain No.45 (LAP) | |

As described above, it was found that the sugar chain No. 45 was a biomarker which could distinguish the most significantly a healthy individual from a rheumatoid patient in any case using a whole serum, a HAP fraction and a LAP fraction, in particular, using the LAP fraction. It was shown by the ROC curve that No. 45 is more excellent biomarker than RF which is an existing biomarker and that the usefulness of No. 45 was the same or more than an anti-CCP antibody which is currently recognized as the most useful antibody.

### Example 3

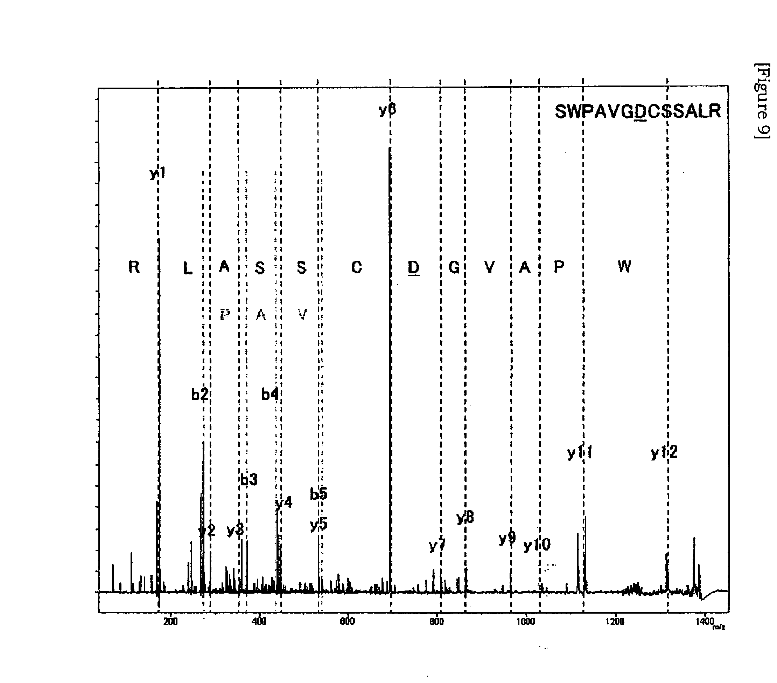

A serum protein was identified in a serum of a healthy individual which was modified by the sugar chain No. 45 which could distinguish the most significantly a healthy individual from a rheumatoid patient.
The serum protein was subjected to trypsin digestion after reduction-alkylation, and then, applied to a concanavarin A column. The non-adsorbed fraction from the concanavarin A column was applied to a Sambucus sieboldiana lectin column and eluted with lactose. The adsorbed fraction to the Sambucus sieboldiana lectin column was fractionated on reverse phase HPLC column and the sugar chain was released by N-glycosidase treatment. The sample which was collected before and after the treatment was analyzed by MALDI-TOF MS to obtain a MALDI-TOF/TOF spectrum in which a shift corresponding to the mass of the sugar chain No. 45 was observed, and then, the spectrum was searched by MASCOT.
An example of identification of the protein having the sugar chain No. 45 was shown in Figure 9. The identified proteins having the sugar chain No. 45 and the peptide sequences of tryptic digest thereof are shown in Table 5 below.

**[Table 5]**

| Protein | Peptide Sequence |
|---|---|
| hemopexin | SWPAVGNCSSALR |
| | NGTGHGNSTHHGPEYMR |
| haptoglobin | NLFLNHSENATAK |
| ceruloplasmin | EHEGAIYPDNTTDFQR |
| | ENLTAPGSDSAVFFEQGTTR |
| | ELHHLQEQNVSNAFLDK |
| clasterin | LANLTQGEDQYYLR |
| kininogen-1 | YNSQNQSNNQFVLYR |
| | ITYSIVQTNCSK |
| | LNAENNATFYFK |
| α1-antichymotrypsin | YTGNASALFILPDQDK |
| apolipoprotein H | VYKPSAGNNSLYR |
| | LGNWSAMPSCK |
| α2-HS glycoprotein | KVCQDCPLLAPLNDTR |
| | VCQDCPLLAPLNDTR |
| | YTGNASALFILPDQDK |
| α1-acid glycoprotein-1 | LVPVPITNATLDQITGK |
| α1-acid glycoprotein-2 | CANLVPVPITNATLDR |
| | LVPVPITNATLDR |
| | QNQCFYNSSYLNVQR |
| zinc α2-glycoprotein | DIVEYYNDSNGSHVLQGR |
| plasma protease C1 inhibitor | NPNATSSSSQDPESLODR |

It was suggested that the proteins which was modified by the sugar chain No. 45 and can distinguish a healthy individual from a rheumatoid arthritis patient are useful as a biomarker of rheumatoid arthritis.

### Example 4

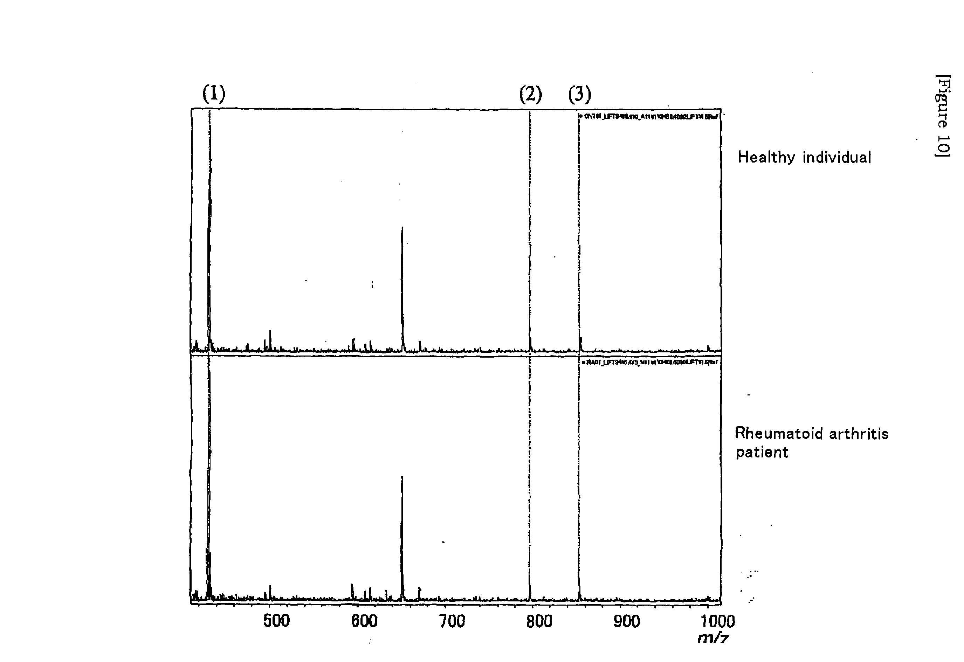

The sugar chain No. 45 which can distinguish the most significantly a healthy individual from a rheumatoid arthritis patient in the sugar chains in a serum from 14 rheumatoid arthritis patients and 11 healthy individuals has the sugar composition of (Hex)₃ (HexNAc)₃ (Deoxyhexose)₁ (NeuAc)₃ + (Man)₃(GlcNAc)₂ (Table 1). It has multiple structural isomers because of diversity of the binding site of a fucose (deoxyhexose). For specifying the structure of No.45 which was useful as a biomarker of rheumatoid arthritis, more detailed structural analysis was conducted.
MALDI-TOF/TOF (MS/MS) analysis using an ion corresponding to the sugar chain No. 45 as a parent ion was conducted with the samples for MALDI-TOF analysis from 14 rheumatoid arthritis patients and 11 healthy individuals which were prepared in Example 1. An example of a fragment pattern obtained as a result of MALDI-TOF/TOF analysis, which was confirmed in a healthy individual and a rheumatoid arthritis patient, was shown in Figure 10. Y ion which is characterized by a structure of a reducing terminal is well observed.
A fragment peak (2) in the figure is a characteristic fragment which is detected only in the case that a fucose binds to a reducing terminal of the sugar chain and a fragment (3) is a fragment which arises frequently in the case that a fucose binds to a branched side chain of the sugar chain. As shown in Figure 10, it was confirmed that a relative intensity of the fragment peaks (2) and (3) tended to differ between a healthy individual and a RA patient, it was suggested that a binding site of a fucose residue in the sugar chain No. 45 differed between a healthy individual and a RA patient. In order to evaluate distribution of the binding site of a fucose residue quantitatively between two groups consisting of the healthy individuals and the RA patients, three Y ions including the fragment peaks (2) and (3) were extracted and normalized as a value relative to a value of the fragment peak (1) which gave a constant value regardless of a binding site of a fucose residue. The results are shown in Table 5. The normalized peak intensity (Table 6-1) and the normalized peak area (Table 6-2) were used as the value.

**[Table 6-1]**

| Normalized peak intensity | | | | |
|---|---|---|---|---|
| Fragment | | Compound (1) | Compound (2) | |
| | Compound (3) | | | |
| m/z | | 433 | 797 | 854 |
| Normal | | | | |
| | average | 1 | 0.142 | 0.095 |
| | s.d. | | 0.038 | 0.018 |
| RA patient | | | | |
| | average | 1 | 0.081 | 0.115 |
| | s.d. | | 0.023 | 0.021 |
| F-test | | | 0.102 | 0.673 |
| t-test | | | *0.0001 | *0.016 |

**[Table 6-2]**

| Normalized peak area | | | | |
|---|---|---|---|---|
| Fragment | | Compound (1) | Compound (2) | |
| | Compound (3) | | | |
| m/z | | 433 | 797 | 854 |
| Normal | | | | |
| | average | 1 | 0.329 | 0.267 |
| | s.d. | | 0.080 | 0.027 |
| RA patient | | | | |
| | average | 1 | 0.200 | 0.299 |
| | s.d. | | 0.063 | 0.034 |
| F-test | | | 0.424 | 0.463 |
| t-test | | | *0.0002 | *0.020 |

(It is a result of the extraction of four Y ions from the MS/MS spectrum and the detailed analysis. All of the data were normalized by setting a value of the fragment peak having a molecular weight 433 (peak (1) in Figure 10) to 1. The normalized peak intensity (Table 6-1) and the normalized peak area (Table 6-2) were used as the value.)
F-test was conducted for confirming if there is difference between the data of a rheumatoid arthritis patient and those of a healthy individual. As a result, it was found that there was no difference between the two groups. Therefore, two sided t-test on the supposition of homoscedasticity was conducted for confirming if there is difference among the above mentioned four Y ions. As a result, it was recognized that there was a significant difference in the fragment peaks (2) and (3). It was proved from this result that the sugar chain No. 45 was a mixture of structural isomers having different binding sites of a fucose. It was further proved that there was a significant difference of an abundance ratio of the structural isomers between the rheumatoid arthritis patients and the healthy individuals, and that in the RA patients, there was a remarkably increased structural isomer where a fucose residue bound to a branched side chain. This is the data which cannot be detected by an existing measurement using only total mass of a sugar chain and a biomarker candidate which is a structural isomer is discovered for the first time by using MS/MS.

In addition, a general formula of a structure of the sugar chain No. 45 is expressed as the following formulae (I) and (II).

### [Chemical formula 1]

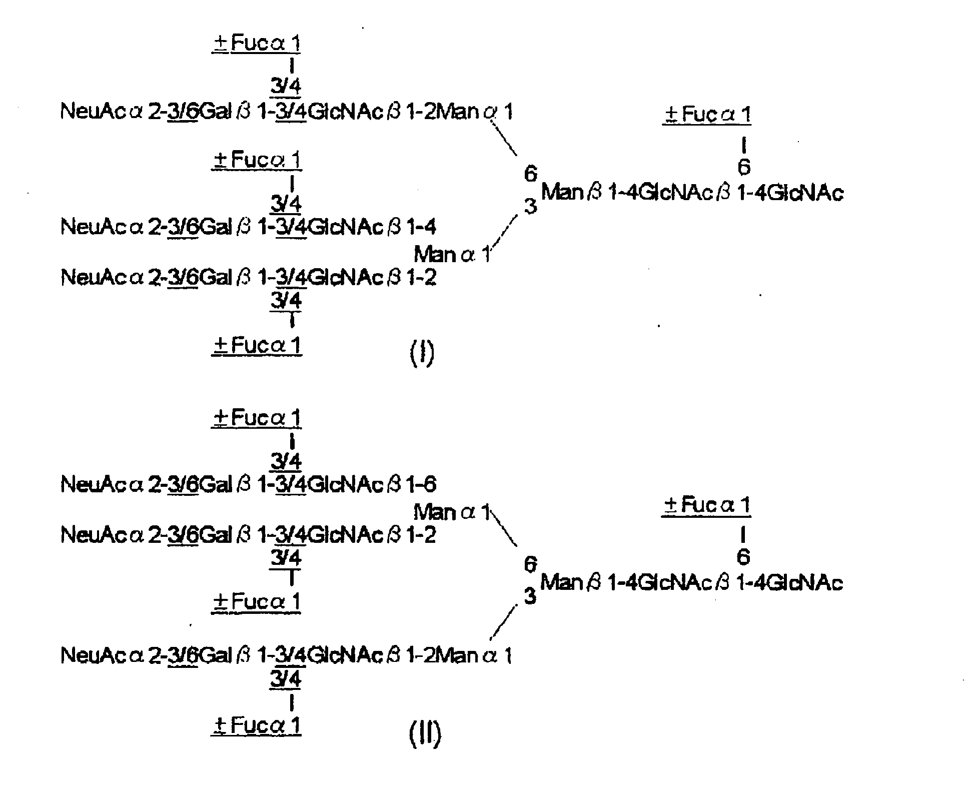

The structural isomers can exist at the underlined sites in the above formulae. That is, NeuAc binds to Gal by either of α2-3 or α2-6 bond and Gal binds to GlcNAc by either of β1-3 or β1-4 bond. Fuc binds to GlcNAc in a branched side chain by either of α1-3 or α1-4 bond or to GlcNAc at reducing terminal by α1-6 bond. However, Fuc binds at only one site among the four binding sites. Not all structures can exist in nature, but the total number of the above structural isomers is 1024(23x23x4x2).

### Example 5

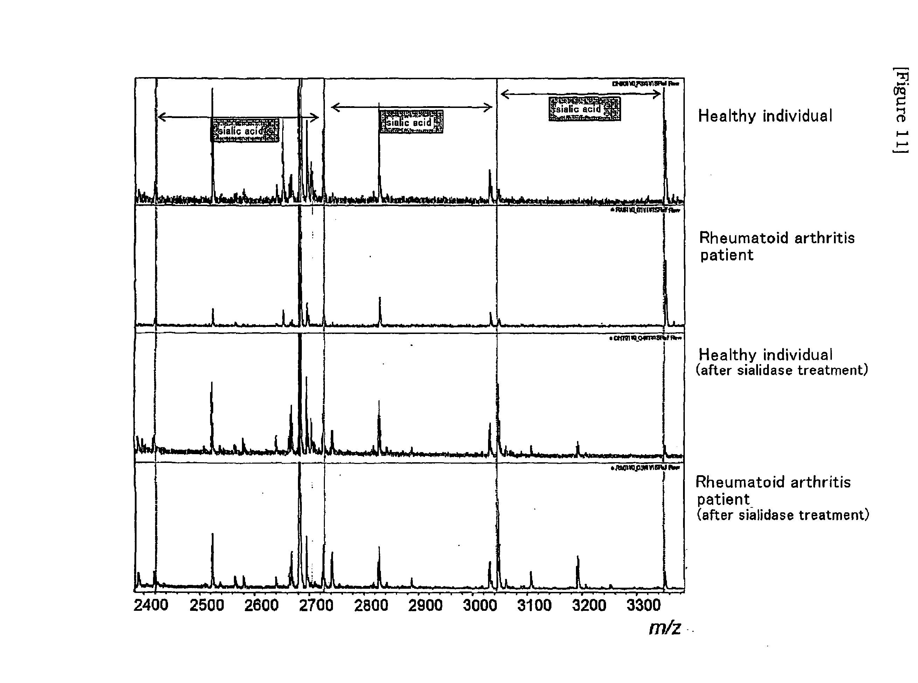

A sugar chain profile of a serum of a patient and a healthy individual after α-2, 3-sialidase digestion was obtained. 100 ml of 200 mM Acetate buffer (pH 4 and 5) was added to 20 µL of a sample which was prepared by releasing the sugar chains from a serum and the pH was adjusted to 5. 2 µL of Neuraminidase (α-2,3-neuraminidase, Macrobdella decora, recombinant in Escherichia coli, Calbiochem) was added to the mixture and the reaction was continued at 37 °C for 16 hours. After the reaction, a sugar chain profile was obtained by Glycoblotting (Figure 11). As a result, a compound in which one sialic acid was cleaved from the sugar chain No. 45 was observed as a product and it was proved that at least one sialic acid was bound by α-2,3-bond.

### INDUSTRIAL APPLICABILITY

A method for diagnosing rheumatoid arthritis which has higher sensitivity and specificity than those of a conventional method, or has complementary superiority is provided by conducting a sugar chain analysis and checking an increase or decrease of a particular sugar chain according to the present invention.

### BRIEF DESCRIPTION OF DRAWINGS

[Figure 1] A MALDI-TOF MS spectrum of the N-linked sugar chains from the rheumatoid arthritis patients and the healthy individuals is shown. The upper stage indicates the sugar chains from the healthy individuals and the lower stage indicates the sugar chains from the rheumatoid arthritis patients.
[Figure 2] An expression level of the sugar chains of the healthy individuals (Ctrl) and the rheumatoid arthritis patients (RA) is shown. The vertical axis is an absolute level of the expressed sugar chains and the horizontal axis is the number of each sugar chain.
[Figure 3] A boxplot which compares a distribution of an expression level of each of the sugar chains No.5, 7, 18, 25 and 29 as well as all the sugar chains between the rheumatoid arthritis patients (RA) and the healthy individuals (Ctrl) is shown. The number in each graph indicates the number of a sugar chain. The vertical axis indicates an expression level of the sugar chain (µM).
[Figure 4] A boxplot which compares a distribution of an expression level of each of the sugar chains No.31, 38, 43 and 45 as well as all the sugar chains between the rheumatoid arthritis patients (RA) and the healthy individuals (Ctrl) is shown. The number in each graph indicates the number of a sugar chain. The vertical axis indicates an expression level of the sugar chain (µM).
[Figure 5] ROC (Receiver Operation Characteristic) curves of RF, anti-CCP antibody and MMP-3 (left graph) and No. 45 (right graph) are shown.

[Figure 6] A boxplot which compares a distribution of an expression level of the sugar chains in HAP between the rheumatoid arthritis patients (RA) and the healthy individuals (Ctrl) is shown. The number in each graph indicates the number of a sugar chain. The vertical axis indicates a relative ratio of the levels.
[Figure 7] A boxplot which compares a distribution of an expression level of the sugar chains in LAP between the rheumatoid arthritis patients (RA) and the healthy individuals (Ctrl) is shown. The number in each graph indicates the number of a sugar chain. The vertical axis indicates a relative ratio of the levels.
[Figure 8] ROC (Receiver Operation Characteristic) curve of the sugar chain No. 45 in LAP is shown.
[Figure 9] An identification example of a protein (hemopexin) having the sugar chain No.45 by MALDI-TOF/TOF is shown. Asparagine (N) which was bound by the sugar chain is converted to Aspartic acid (D) along with release of the N-linked sugar chain by N-glycosidase treatment.
[Figure 10] MS/MS spectrum of the sugar chain No. 45 of a healthy individual and a rheumatoid arthritis patient is shown. The composition of the fragment ions (1), (2) and (3) indicates (1) reagent of reducing terminal, (2) reagent+N-acetylglucosamine+fucose and (3) reagent+N-acetylglucosamine+N-acetylglucosamine.
[Figure 11] A sugar chain profile after α-2, 3-sialidase treatment is shown. A cleavage of sialic acid is observed after the treatment.

## Claims

1. A method for diagnosing rheumatoid arthritis using an expression level of one or more sugar chains as an index, wherein the sugar chains are selected from a group consisting of No. 1, 7, 8, 10, 11, 17, 18, 21, 24, 25, 27, 29, 31, 38, 41, 43, 45, 46 and 48.

2. The method using an expression level of the sugar chain No. 45 as an index.

3. The method according to claim 1 or 2, wherein a fucose in composition of the sugar chain 45 is attached to a side chain.

4. The method according to any of claims 1 to 3, wherein an expression level of a sugar chain having the composition of No. 45 and having sialyl Lewis x (NeuNAcα2→3Galβ1→4(Fucα1→3)GlcNAc) or sialyl Lewis a (NeuNAcα2→3Galβ1→3(Fucα1→4)GlcNAc) as an epitope is used as an index.

5. The method according to any of claims 1 to 4, wherein the index is an expression level of the sugar chains in a serum.

6. Use of one or more sugar chains as a marker for rheumatoid arthritis, wherein the sugar chains are selected from a group consisting of No. 1, 7, 8, 10, 11, 17, 18, 21, 24, 25, 27, 29, 31, 38, 41, 43, 45, 46 and 48.

7. Use of the sugar chain No. 45 as a marker for rheumatoid arthritis.

8. The use according to claim 6 or 7, wherein a fucose in the composition of the sugar chain 45 is attached to a side chain.

9. The use of an expression level of a sugar chain having the composition of No. 45 and having sialyl Lewis x (NeuNAcα2→3Galβ1→4(Fucα1→3)GlcNAc) or sialyl Lewis a (NeuNAcα2→3Galβ1→3(Fucα1→4)GlcNAc) as an epitope according to any of claims 6 to 8.

10. A method for screening a rheumatoid arthritis sample comprising the following processes:
(i) analyzing the sugar chains in a test sample; and
(ii) comparing an expression level of one or more sugar chains of a sample from a healthy individual with those of the test sample, wherein the sugar chains are selected from a group consisting of No. 1, 7, 8, 10, 11, 17, 18, 21, 24, 25, 27, 29, 31, 38, 41, 43, 45, 46 and 48.
